# EUROPEAN PATENT APPLICATION

(11) **EP 2 123 220 A1**
(43) Date of publication of application: **25.11.2009**
(21) Application number: 08104029.7
(22) Date of filing: 20.05.2008
(51) Int. Cl.: A61B 5/107, A61B 1/227, A61B 19/00

(54) **A probe and coil fixed thereto for establishing the spatial location of a probe body and a method of fixedly position a magnetic generating means to a probe body and a system for obtaining geometrical data related to a cavity**

(71) Applicant: Oticon A/S, 2765 Smørum (DK); Widex A/S, 3500 Værløse (DK)
(72) Inventor: Petersen, Henrik Poulin, DK-2765, Smørum (DK)

(57) **Abstract**

Disclosed is a probe for obtaining geometrical data related to a cavity, the probe comprising a probe body comprising a first coupling means; at least one magnetic field generating device comprising a support member and a means for generating a magnetic field; wherein said means for generating a magnetic field being connected to the support member so as to fix a position of said means for generating a magnetic field relative to said support member and wherein said support member comprises a second coupling means configured to engage the first coupling means so as to connect the at least one magnetic field generating device to said probe body..

Thereby is achieved to improve positional accuracy of geometrical data related to an internal surface of a cavity.

## Description

### Field of the invention

The present invention is related to a method of fixedly position a magnetic generating means to a probe or similar body, and a system for obtaining geometrical data related to a cavity, such as for example the ear and ear canal of the human body.

### Background of the invention

In order to make a shell which fits a cavity such as for example the ear and ear canal of the human body, an apparatus enabling generation of a data mapping of the internal surface of the ear and ear canal may be utilized, so that 3-dimensional data or a digital model of the internal surface of the ear and ear canal can be obtained. Such a 3-dimension model can be used to produce the shell, which may have the exact shape of the canal and the shell may form the basis for an e.g. In-The-Ear (ITE) or Completely-In-The-Canal (CIC) hearing aid. Also ear moulds or shells for other purposes such as a hearing protection or for headsets may be produced from the data model. The shell can be produced on the basis of the data model in different ways, such as by recent developed rapid prototyping methods or by well known machining, e.g. in a Computer Numerically Controlled (CNC) machining centre.

It remains a problem to improve the position accuracy of devices for data mapping of internal surfaces such as the ear and/or ear canal of a person. A lack of positional accuracy in geometrical 3-D data obtained from the apparatus generating a data mapping may, for example, give rise to discomfort to the person using the shell e.g. a CIC hearing aid. This problem is also at hand in connection with other tracking systems, where the location of a body can only be determined with high precision if magnetic field generating elements are positioned precisely thereto and in strictly orthogonal relationship.

### Summary

Disclosed is a probe and a coil fixed thereto system for establishing the spatial location of the probe wherein the probe and coil comprises a probe body with a first coupling means; at least one magnetic field generating device comprising a bobbin and a coil for generating a magnetic field; wherein the coil for generating a magnetic field is connected to the bobbin so as to fix a position the coil for generating a magnetic field relative to the bobbin and wherein the bobbin comprises a second coupling means configured to engage the first coupling means so as to connect the bobbin and coil to the probe body.

Consequently, it is an advantage that the bobbin (e.g. a base plate 402) comprises a second coupling means adapted to engage the first coupling means of the probe (e.g. a probe comprising a distal light-emitting end) because it enables accurate positioning of the bobbin with respect to the probe. Additionally, it is an advantage that the coil for generating a magnetic field is connected to the bobbin so as to fix a position of the coil relative to the bobbin because it enables accurate placement of the coil with respect to the probe. Thereby an accurate position of the magnetic field generated by the means coil is obtained. This accurate positioning is with respect to, for example, the probe and/or a distal light-emitting end of the probe.

For example, the distal light-emitting end of the probe obtains the geometrical data. Accurate placement of the coil with respect to the distal light-emitting end and a constant distance between the coils enables accurate determination of the position of the geometrical data, because the position of the geometrical data is determined with respect to the location of the coil, and with respect to distal light-emitting end distance and with respect to the coil's distance to, for example, an external sensor detecting at least one magnetic field generated by said coil. Thus, an improved determination of the position of geometrical data with respect to e.g. an external sensor may be obtained by placing the coil with a fixed position on a base plate and the base plate with a fixed position on the probe.

Further, when the probe comprise two or more spaced apart coils for generating the magnetic field in one direction, these coils should be aligned to ensure that the magnetic fields are also aligned both with respect to angle and spatial placement. This is especially important when two orthogonal fields is desired, as mis-allignment will inevitably cause cross coupling between the two orthogonal fields. If the coils are ldelly aligned having fields in precisely right angles with respect to each other, the two fields will be independent, but if one coil is mis-alligned, this coil will be influenced by the orthogonally placed other magnetic field and vice-versa.

The probe is not as such essential to the invention, as the means for locating the coil or coils at a fixed position may be used at other devices when there is a need to establish the location thereof.

In an embodiment, one of the first and second coupling means comprises a protrusion and the other one of the first and second coupling means comprises a hole adapted to receive said protrusion.

In a further embodiment, the size of the hole adapted to receive the protrusion is identical to or larger than the size of the protrusion.

An advantage of this embodiment is that the first and second coupling means may fit together to a high degree, e.g. by frictional engaging each other, thereby increasing the accuracy by which the at least one magnetic field generating device is placed on the probe.

In a further embodiment, the size of a hole in the first coupling means are greater than the size of a corresponding protrusion of the second coupling means and/or the size of a protrusion in the first coupling means are smaller than the size of a corresponding hole in the second coupling means.

An advantage of this embodiment is that production errors in the first and second coupling means may be encompassed.

In a further embodiment, the first coupling means comprises a first plurality of protrusions and/or a second plurality of holes and wherein the second coupling means comprises the first plurality of corresponding holes and/or the second plurality of corresponding protrusions.

An advantage of this embodiment is that at least two protrusions and/or two holes are in the probe and the at least one magnetic field generating device which enables accurate placement of a means for generating a magnetic field where the magnetic field generated is not rotational symmetric around the at least two holes and/or at least two protrusions of the probe. Thereby, a non-rotational symmetric magnetic field generated by the means for generating a magnetic field may be placed as demanded e.g. by probe design.

In a further embodiment, the probe comprises at least bobbins with each their coil adapted to generate respective magnetic fields in substantially the same direction.

An advantage of this embodiment is that two means for generating a magnetic field e.g. two coils may be oriented such that their respective magnetic fields are oriented in the same direction and thus add up to a resulting magnetic field which is greater than the respective magnetic fields.

The present invention relates to different aspects including the method described above and in the following, and corresponding methods and system, each yielding one or more of the benefits and advantages described in connection with the first mentioned aspect, and each having one or more embodiments corresponding to the embodiments described in connection with the first mentioned aspect and/or disclosed in the appended claims.

In particular, disclosed herein is a method of fixedly position a magnetic generating device to a probe body wherein the probe comprises a first coupling means and at least one magnetic field generating device wherein the magnetic field generating device comprises a bobbin with a second coupling means and a coil for generating the magnetic field; wherein the method comprises, connecting the coil for generating a magnetic field to the bobbin so as to fix a position of said coil for generating a magnetic field relative to said bobbin and connecting the at least one magnetic field generating device to the probe body by engaging the second coupling means of the bobbin to the first coupling means of the probe.

Further in particular, disclosed herein is a system for obtaining geometrical data related to a cavity, the system comprising at least one probe body with magnetic generating means fixedly positioned thereon according to the present invention and the system further comprising at least a plurality of magnetic sensor for detecting the at least one probe.

### Brief description of the drawings

The above and/or additional objects, features and advantages of the present invention, will be further elucidated by the following illustrative and nonlimiting detailed description of embodiments of the present invention, with reference to the appended drawings, wherein:
Fig. 1 shows a schematic view of an example of an apparatus for obtaining geometrical data relating to the internal surface of a cavity.
Fig. 2 shows a schematic view of another example of an apparatus for obtaining geometrical data relating to the internal surface of a cavity.
Fig. 3 shows a sectional view of the distal end of a probe of an apparatus for obtaining geometrical data relating to the internal surface of a cavity.
Fig. 3a shows a cross sectional view of a distal light-emitting probe end comprising a number of coils.
Fig. 3b shows an image of a probe comprising four coils placed in the distal light-emitting end of the probe.
Fig. 4a shows a distal light-emitting end portion of a probe comprising at least one protrusion.
Fig. 4b shows a distal light-emitting end portion of a probe comprising at least one cut-out.
Fig. 4c shows a coil housing comprising a coil, a base plate and a cover.
Fig. 5a shows an example of how two coils in two coil housings of a probe may be connected in series.
Fig. 5b shows an example of the magnetic fields generated by two coils connected in series and mounted on a probe.
Fig. 6 shows an example of a series resonance circuit.
Fig. 7 shows an example of a probe containing a printed circuit board contained in a shielding box.
Fig. 8 shows a side view of a human ear and illustrating the use of the apparatus described herein.

### Detailed description

In the following description, reference is made to the accompanying figures, which show by way of illustration how the invention may be practiced.

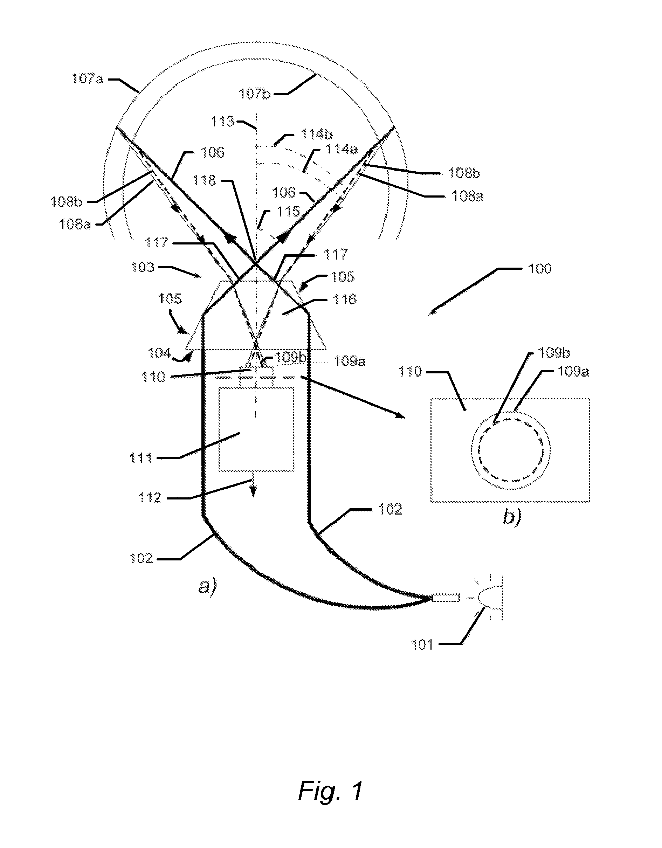

Fig. 1 a shows a schematic view of an example of an apparatus for obtaining geometrical data relating to the internal surface of a cavity. The apparatus, generally designated 100, comprises a distal end portion 103 including an optical system 116 for emitting and receiving light, a light source 101 and light guides 102 for directing the light from the light source 101 to a rear surface 104 of the optical system 116. The optical system 116 may comprise one or more lenses and/or one or more reflecting surfaces 105 for directing the light from the light guides 102 as one or more beams 106 to the internal surface 107a, b of a cavity.

The light beams 106 are emitted at an acute angle 115 relative to the optical axis 113 of the optical system 116 which also defines the direction of insertion of the distal end portion 103 into the cavity. The light beams 106 are emitted from respective exit positions 117 radially displaced from the optical axis. Furthermore, the exit position 117 and the position where the light beam intersects with the cavity wall 107a, b, are positioned on opposite sides of the optical axis 113. The light beams 106 thus cross the optical axis. Furthermore, the light beams 106 intersect at a point 118 in front of the probe; in the example of fig. 1 the beams intersect with each other on the optical axis.

In the example of fig. 1, two light guides 102 are shown. However, it will be appreciated that generally a different number of light guides may be used, e.g. 3, 4, 5, 6, or even a larger number of light guides. In some embodiments a uniform illumination over a full circumference is achieved by providing a relatively large number, e.g. between 60 or 80, fibres. The fibres are divided into bundles, e.g. 6 bundles, and each bundle is illuminated with a respective light source. This gives the opportunity to make an automatic control of the emitted power at different sections of the probe. This is advantageous as it allows staying in the dynamic range of the light detector, when measuring surfaces at different distances in different sections at the same time.

For example, the light guides may be arranged such that the emitted light beams 106 intersect with each other as shown in figure 1, so as to define a double cone where the point of intersection 118 defines the apex of the cone. In the example of fig. 1, the beams 106 intersect each other on the optical axis.

The emitted light beams 106 are reflected from the internal surface 107a, b of the cavity and at least a portion of the reflected light will be reflected back in the direction of the distal end portion 103 as indicated by reflected beams 108a, b in fig. 1. The optical system 116 of the distal end portion 103 thus also functions as a light receiving system and receives the reflected light 108a, b from the cavity walls 107a, b. The optical system 116 directs the received light 108a, b towards a light sensitive element 110, e.g. an array of light sensitive elements such as a CCD or another position sensitive light detector.

Generally, if the CCD is a colour sensitive CCD element, colour information may be used when analyzing the light reflected from the surface of an ear canal. If white light is used, it is possible to determine the relative content of red, green and blue light in the received signal, and thereby foreign objects such as earwax may be identified. This is because earwax will reflect the light in other wavelength ranges than the naked skin of the ear canal.

Furthermore, the detected signal from the CCD may be displayed on a display, via an eyepiece, or the like, as a received image, thus allowing the probe described herein to be used in a fashion similar to an endoscope. Such an image may be valuable for the person conducting an ear scan, e.g. for a visual inspection of the measured cavity and/or so as to provide a visual control as to how close the probe is to the end wall, e.g. the tympanic membrane.

The light source 101 may be any suitable light source, e.g. a one or more light emitting diodes (LED) or diode lasers. LEDs provide a low noise level as they avoid noise from speckle, while diode lasers provide a high output power.

In fig. 1, the cavity surface 107a, b is shown as an example in a first distance at 107a from the tip of the probe and in a second distance at 107b closer to the tip of the probe. Light reflected from the surface at 107a will enter the optical system 116 as light 108a at an incident angle 114a relative to the optical axis, while light reflected from the surface at 107b will enter the optical system 116 as light 108b at an incident angle 114b relative to the optical axis. Consequently, the optical system 116 directs the incoming light 108a and 108b to respective positions 109a and 109b on the light sensitive element 110, thereby allowing the determination of the distance to the points 107a and 107b in the direction of the emitted light 106 from the position of the detected light on the light sensitive element 110. Fig. 1b illustrates positions of constant distance on the light sensitive area of the detector 110 for two different distances 109a and 109b respectively.

The apparatus 100 further comprises a signal analysis circuit 111 which generates an output signal 112, e.g. an analogue signal or a digital data signal. In some embodiments, the output signal is indicative of an intensity distribution of the detected light across the light sensitive area 110 of the detector. Alternatively or additionally, the signal analysis circuit may perform additional signal processing steps, e.g. including the actual distance calculation based on an incident angle determined from the detected locations 109a and 109b. The distance may be calculated by means of a conventional triangulation resulting in a distance from the probe to the locations where the beams 106 intersect with the cavity wall 107a,b, i.e. a distance in a direction having a component in a radial direction from the optical axis 113. Alternatively, the distance calculation and/or further signal processing may be performed by a separate signal/data processing unit, e.g. on a computer such as a PC to which the apparatus 100 may be connected.

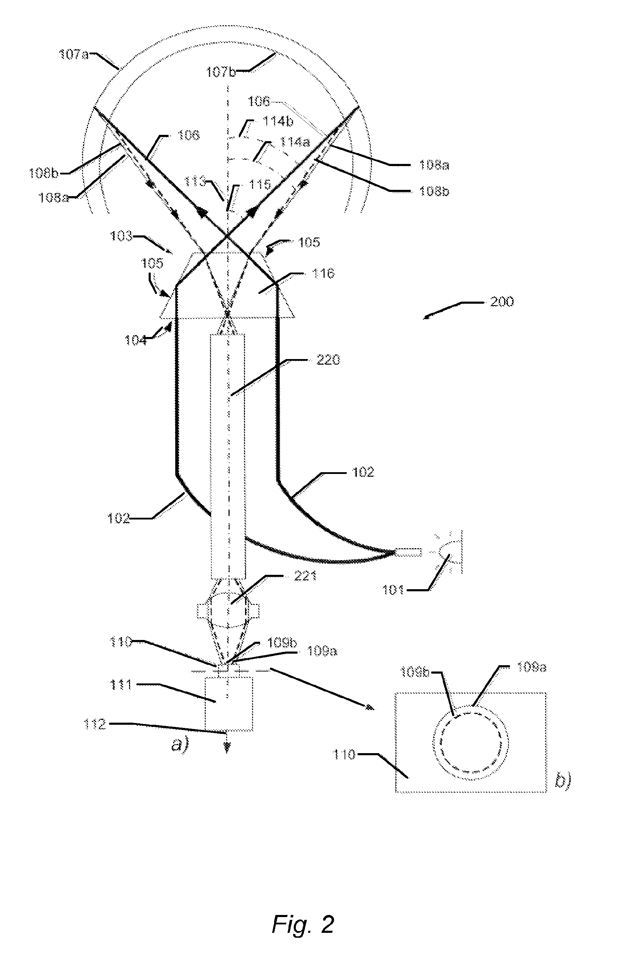

Fig. 2 shows a schematic view of another example of an apparatus, generally designated 200, for obtaining geometrical data relating to the internal surface of a cavity. The apparatus 200 is similar to the apparatus described in connection with 100, and will therefore not be described in detail again here. However, while the light sensitive element 110 of the apparatus shown in fig. 1 was arranged adjacent to the distal end portion 103, such that the reflected light is captured at the distal end of the probe, the light sensitive element 110 of the apparatus 200 is arranged remote from the distal end 103. To this end, the optical system 116 at the distal end portion 103 directs the reflected light into the distal end of a light guide 220, which in the following will be referred to as an imaging guide, the imaging guide 220 thus directs the received light, e.g. via a further lens or lens system 221 to the light sensitive element 110.

While the apparatus of fig. 100 provides a simpler construction, the apparatus 200 does not require a light sensitive element, e.g. a CCD which is small enough to be mounted at the tip of the probe, which is going to enter the cavity. For example, the light guides 102 and the imaging guide 220 may be arranged in a flexible tube connecting the distal end portion 103 with a proximal unit including the light source 110, the detector 110, and, optionally a signal processing unit 111.

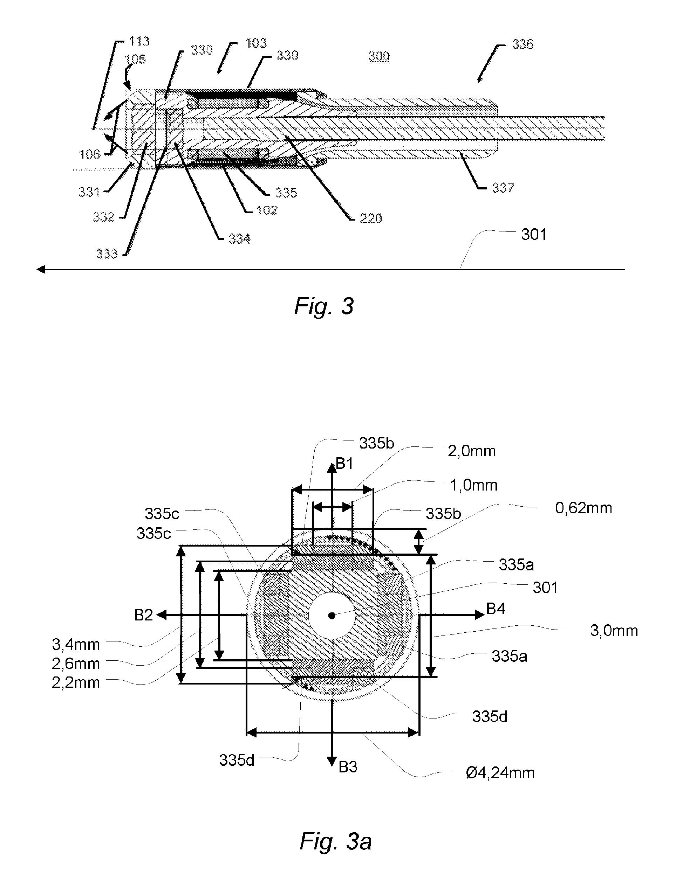

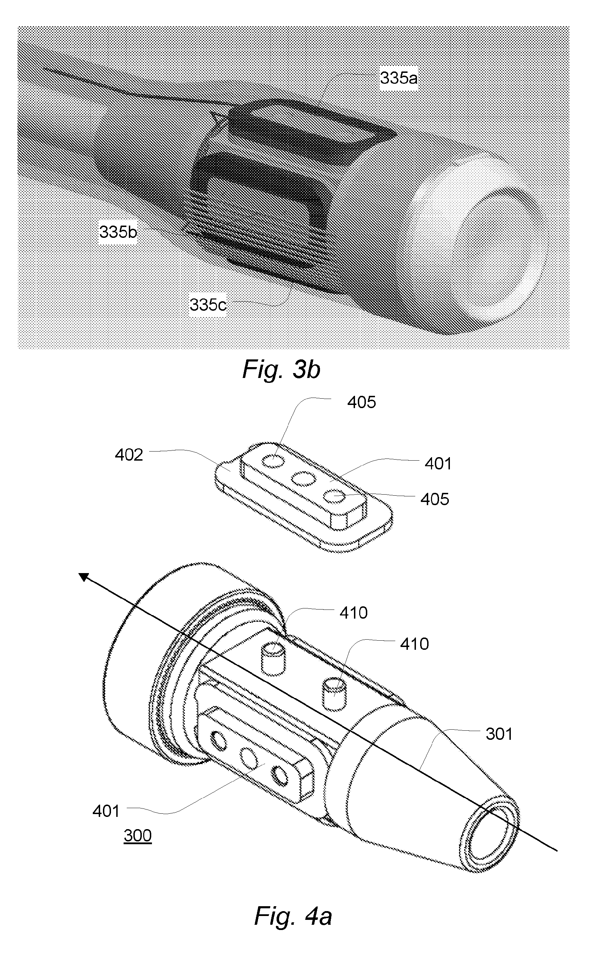

Fig. 3 shows a sectional view of the distal end of a probe of an apparatus for obtaining geometrical data relating to the internal surface of a cavity. The probe, generally designated 300, has a distal light-emitting end portion 103 and a rod portion 336, which connects the distal portion to a proximal part (not explicitly shown). The rod portion 336 comprises a flexible pipe 337, a set of light guides 102 and an image guide 220. The image guide 220 is placed centrally in the pipe 337, and the light guides 102 are arranged between the pipe 337 and the image guide 220. Near the tip of the probe the image guide 220 and the light guides 102 are connected to a bushing 330 surrounded by an outer tube 339.

The probe further comprises an optical system including an annular lens 331 arranged at the bushing 330 to capture the light emitted from the light guides 102 and to direct the light towards a cavity wall, e.g. as a collimated or focussed light beam 106, at an acute angle from the longitudinal axis of the probe which also defines the optical axis 113 and the direction of insertion.

Light reflected from the cavity wall will enter the tube 339 through a central receiving lens 332 of the optical system. From the lens 332 the light is directed via an aperture 333 and a further imaging lens 334 towards a surface of the image guide 220. The aperture 333 increases the depth of field and prevents stray light from reaching the sensor. The light received on the surface of the image guide 220 is transmitted through the image guide 220, and will appear at the other end thereof. Here the image is captured by a CCD array (not shown). The signal from the CCD is transferred to a signal processing unit for further processing in order to calculate the distance from the probe to the canal wall. This is done by a triangulation method well known as such in the art.

In general, even though the light 106 directed towards the cavity wall may be unfocussed or uncollimated, the use of focused or collimated light provides better contrast and thus results in a more precise detection of the distance between the probe and the cavity wall.

The probe 300 further comprises one or more coils 335 used to generate a magnetic field, which is picked up by sensors arranged outside the cavity so as to determine the position of the probe relative to the external sensors. Thus, when the sensors are arranged in a fixed spatial relationship to the cavity, a signal/data processing unit can compute spatial coordinates of positions on the inner surface of the cavity from the optical distance measurements relative to the probe and from the position measurements of the position of the probe relative to the external sensors.

The probe 300 may comprise one or more coils 335 such as for example one coil or two coils or three coils or four coils or any number of coils 335 greater than or equal to one. The one or more coils may, for example, be placed such that the magnetic field generated by each of the one or more coils is directed in one or more directions lying in a plane perpendicular to a longitudinal axis 301 of the probe 300.

Fig. 3a shows a cross section of a probe 300 comprising four coils 335a - 335d, where said cross section is made perpendicular to the longitudinal axis 301 of the probe 300 and through the four coils 335a - 335d. The coils 335a - 335d are placed such that the magnetic field generated by each of the four coils is directed in a plane perpendicular to the longitudinal axis 301 of the probe 300 such as for example indicated by the magnetic field vectors B1 - B4 generated by the coils 335a - 335d, respectively. Additionally, the coils 335a - 335d may be placed such that an angle of 90 degrees (or substantially 90 degrees e.g. 88 - 92 degrees) may be between the magnetic field vectors B1 and B2 of coils 335b and 335c, respectively, 90 degrees (or substantially 90 degrees e.g. 90±0.25 degrees) may be between the magnetic field vectors B2 and B3 of coils 335c and 335d, respectively, 90 degrees (or substantially 90 degrees e.g. 90±0.25 degrees) may be between the magnetic field vectors B3 and B4 of coils 335d and 335a, respectively, and 90 degrees (or substantially 90 degrees e.g. 90±0.25 degrees) may be between the magnetic field vectors B4 and B1 of coils 335a and 335b, respectively.

If the probe 300 comprises more than one coil for generating a magnetic field, such as for example four coils generating magnetic fields vectors B1 - B4, respectively, then the four coils may be identical to each other e.g. generating identical magnetic fields when a current is passed through the coils and/or comprising the same number of turns and/or comprising the same wire diameter size etc. Alternatively, the four coils may be different from each other e.g. generating different magnetic fields when a current is passed through the coils and/or comprising a different number of turns and/or comprising the different wire diameter size etc. Alternatively, the probe may comprise a number of identical coils, e.g. two identical coils, and a number of different coils, e.g. two coils different from each other and different from the two identical coils.

As shown in figure 3, the one or more coils 335 may be positioned in the distal light-emitting end portion 103 of the probe 300.

Fig. 3b shows an image of a probe 300 comprising four coils 335a - 335d (of which three are visible in the image). The four coils may, for example, generate four magnetic fields.

Fig. 4a shows a distal light-emitting end portion 103 of a probe 300 comprising at least one protrusion 410 and at least one coil bobbin 401. For example, the probe 300 may comprise two protrusions 410 one coil bobbin 401 for each coil 335 to be mounted on the probe 300. For example, if four coils 335 are to be mounted on the probe 300, then the probe 300 may comprise four times two protrutions 410.

The at least one protrusion 410 may be constructed as an object extending outwards from the probe 300 e.g. extending vertically outwards in a direction perpendicular to the longitudinal axis 301 of the probe 300. The at least one protrusion 410 may have any geometrical form such as for example cylindrical as indicated in Fig. 4a. Alternatively, the at least one protrusion 410 may have the geometrical form of a box or a cone or any other geometrical form suitable for having a bobbin 401 with coil 335 or a coil housing with a substantially corresponding cut-out (or hole) mounted on it.

The bobbin 401 may comprise a base plate 402 (a support member)

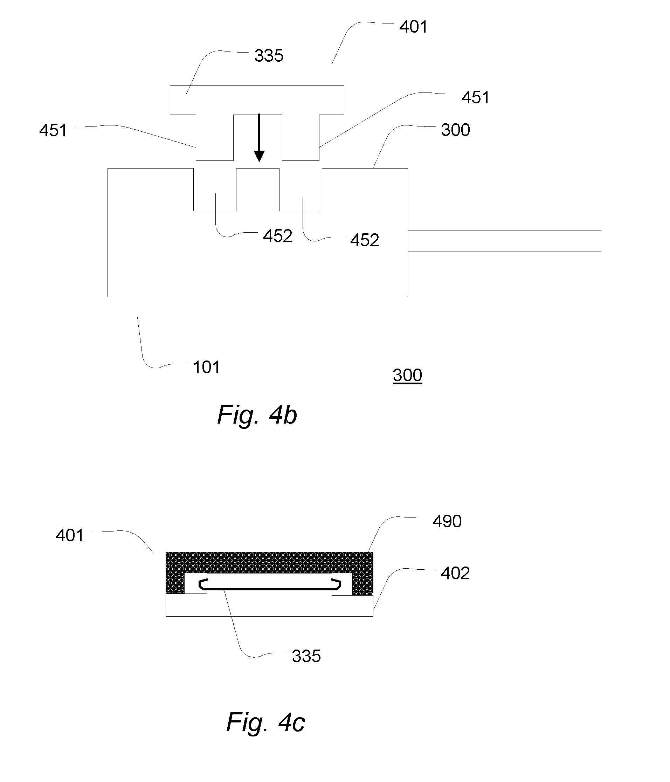

The base plate 402 may, for example, comprise a cylindrical promontory, around which a coil 335 is wound as e.g. shown in Fig. 4c. Alternatively, the coil 335 may be pre-wound and have an internal diameter enabling the coil 335 to be mounted around the promontory of the base plate 402. Additionally, a cover 490 may be cast around the coil 335 for example in order to protect the coil from damage and/or to enable easier handling of the bobbing 401. Alternatively, the cover 490 may be pre-cast and subsequently mounted around the coil

The bobbin 401 including a coil 335 may further include a cut-out 405 or a hole 405 of similar geometrical form as the protrusion 410. The cut-out 405 may, for example, be in the base plate 402. For example, if the probe 300 comprises a protrusion 410 which is cylindrical, then the bobbin 401 may comprise a cylindrical cut-out 405 or a cylindrical hole 405 enabling the bobbin 401 to be placed onto the probe such that the cylindrical cut-out 405 (or cylindrical hole 405) of the bobbin 401 is penetrated and filled by the protrusion 410.

The cut-out 405 (or the hole 405) in the bobbin 401 may be extending all the way through the bobbin 401 e.g. through the base plate 402 and the cover 490. Alternatively, the cut-out 405 (or the hole 405) may be of a type not extending all the way through the bobbin 401 e.g. only in the base plate.

If, for example, the probe 300 comprises two protrusions 410 which are cylindrical, then a bobbin 401 may comprise two cylindrical holes 405 enabling the bobbin 401 to be placed onto the two protrusions 410. If, for example, the probe 300 comprises two protrusions, e.g. a cylindrical and a conical protrusion, the a bobbin 401 may comprise a corresponding cylindrical and a conical cut-out enabling the bobbin to be placed onto the cylindrical and the conical protrusions 410.

In an embodiment, the dimensions of the volume of the one or more holes 405 in a bobbing 401 may correspond to or substantially correspond to the dimensions of the volume of the at least one protrusion 410 onto which the bobbing 401 is to be mounted or is mounted.

For example, if a bobbing 401 is to be mounted onto two protrusions 410 on a probe 300, each of the protrusions 410 having a cylindrical shape with a volume of dimension Height x Diameter and the two protrusions 410 being separated by a distance Separation, e.g. measured from center to center of the two protrusions 410, then the bobbing 401 to be mounted onto the two protrusions 410 may comprise two cylindrical holes 405 each with a dimension of Height x Diameter and the two holes 405 being separated by the distance Separation such that the bobbing 401 may be fitted onto the protrusions 410.

In an additional or alternative embodiment, the holes 405 of the bobbing 401 may have a volume slightly larger than the volume of the protrusions 410 in order to ensure that the bobbing 401 may be placed onto the protrusions 410 e.g. in order to encompass production uncertainties of the protrusions 410. For example, if the dimensions of two cylindrical protrusions 410 are Height x Diameter, then the dimensions of the holes 405 of the bobbing 401 to be mounted onto the protrusions 410 may be (Height + ε) x (Diameter + ε). Alternatively, one of the dimensions of the bobbing 401 and volume of the hole 405 may be slightly larger than the corresponding protrusion 410 dimension e.g. if the dimensions of two cylindrical protrusions 410 are Height x Diameter, then the dimensions of the holes of the bobbing 401 to be mounted onto the protrusions 410 may be Height x (Diameter + ε). In the above, ε may be a real number greater than zero representing a distance. For example, ε may be chosen in the range of 0.01 mm - 0.05mm.

By mounting the one or more bobbins 401 on the at least one protrusion 410 by means of said holes 405 or cut-outs 405 in said one or more bobbins 401, e.g. by mounting four bobbins 401 each comprising two holes 405 on four times two protrusions 410, enables the probe 300 to ensure good alignment of the coils 335 with respect to the probe 300.

The coil winding has to be done so the wires are arranged with equal number of turns in each layer. Also the placement of the wires has to be done nicely and tight to the coil bobbin. The reason for this, is to achieve the highest possible symmetrical magnetic system when placed on the probe head, this will lead to a minimum of cross coupling. A safe way to achieve this is to wind the coil directly onto the bobbin.

Fig. 4b shows an additional or alternative embodiment in which a distal light-emitting end portion 103 of a probe 300 comprises at least one cut-out 452. For example, the probe 300 may comprise two cut-outs 452 for each coil 335 to be mounted on the probe 300. For example, if four coils 335 are to be mounted on the probe 300, then the probe 300 may comprise four sets of two cut-outs 452 e.g. placed in a plane perpendicular to the longitudinal axis 301 of the probe 300 and with 90 degrees or substantially 90 degrees (e.g. 88 - 92 degrees) between each of the sets. Alternatively, the probe may comprise any number of cut-outs 452 greater than or equal to one for each coil 335 to be mounted on the probe 300.

The at least one cut-out 452 may be constructed as a hole extending inwards in the probe 300 e.g. in a direction perpendicular to the longitudinal axis 301 of the probe 300. The at least one cut-out 452 may have any geometrical form such as for example a cylindrical hole as indicated in Fig. 4b. Alternatively, the at least one cut-out 452 may have the geometrical form of a box or a cone or any other geometrical form cut-out from the probe 300.

Each of the one or more coils 335 may be included in a respective bobbing 401. The bobbing 401 (a magnetic field generating device) may, for example, comprise a base plate 402 (a support member) and a coil 335 (means for generating a magnetic field).

The base plate 402 may, for example, comprise a promontory e.g. a cylindrical promontory, around which a coil 335 may be wound as e.g. shown in Fig. 4c. Alternatively, the coil 335 may be pre-wound and have an internal diameter enabling the coil 335 to be mounted around the promontory of the base plate 402. Additionally, a cover 490 may be cast around the coil 335 for example in order to protect the coil from damage and/or to enable easier handling of the bobbing 401. Alternatively, the cover 490 may be pre-cast and subsequently mounted around the coil

Further, the bobbing 401 may further include one or more protrusions 451 of similar geometrical form corresponding to cut-outs 452. For example, if the probe 300 comprises a cut-out 452 which is cylindrical, then a bobbing 401 may comprise a cylindrical protrusion 451 enabling the protrusions 451 of the bobbing 401 to be placed into the cut-out 452 such that the cylindrical protrusion 451 of the bobbing 401 penetrates and fills the cylindrical cut-out 452 of the probe.

Alternatively, if the probe comprises a conical cut-out 452, then the bobbing 401 may comprise a conical protrusion 451 enabling the protrusions 451 of the bobbing 401 to be placed into the cut-out 452 such that the conical protrusion 451 of the bobbing 401 penetrates the conical cut-out 452.

The cut-out 451 in the probe 300 may be extending all the way through the probe 300. Alternatively, the cut-out 451 in the probe 300 may be of a type not extending all the way through the probe 300.

If, for example, the probe 300 comprises two cut-outs 451 which are cylindrical, then a bobbing 401 may comprise two cylindrical protrusions 451 enabling the protrusions 451 of the bobbing 401 to be placed into the two cut-outs 452. If, for example, the probe 300 comprises two cut-outs 452, e.g. a cylindrical and a conical cut-out, the bobbing 401 may comprise a corresponding cylindrical and a conical protrusion enabling the protrusions 451 of the bobbing 401 to be placed into the cylindrical and the conical cut-outs 452.

In an embodiment, the dimensions of the volume of the one or more protrusions 451 in a bobbing 401 may correspond to or substantially correspond to the dimensions of the volume of the at least one cut-out 452 into which the protrusions 451 of the bobbing 401 is to be mounted or is mounted.

For example, if two protrusions 451 of a bobbing 401 are to be mounted into two cut-outs 452 in a probe 300, each of the cut-outs 452 having a cylindrical shape with a volume of dimension Height x Diameter and the two cut-outs 452 being separated by a distance Separation, e.g. measured from center to center of the two cut-outs 452, then the two protrusions 451 of the bobbing 401 to be mounted into the two cut-outs 452 may comprise two cylindrical protrusions 451 each with a dimension of Height x Diameter and the two cylindrical protrusions 451 being separated by the distance Separation, e.g. measured from center to center of the protrusions 451, such that the two protrusions of the bobbing 401 may be fitted into the cut-outs of the probe 300.

In an additional or alternative embodiment, the protrusions of the bobbing 401 may have a volume slightly smaller than the volume of the cut-outs 452 in order to ensure that the protrusions 451 of the bobbing 401 may be placed into the cut-outs 452 of the probe 300 e.g. in order to encompass production uncertainties of the cut-outs 452. For example, if the dimensions of two cylindrical cut-outs 452 are Height x Diameter, then the dimensions of the two protrusions 451 of the bobbing 401 to be mounted into the cut-outs 452 may be (Height - ε) x (Diameter - ε). Alternatively, one of the dimensions of the bobbing 401 protrusion volume may be slightly smaller than the corresponding cut-out 452 dimension e.g. if the dimensions of two cylindrical cut-outs 452 are Height x Diameter, then the dimensions of the two protrusions of the bobbing 401 to be mounted into the cut-outs 452 may be Height x (Diameter - ε). In the above, ε may be a real number greater than zero representing for example a distance. For example, ε may be chosen in the range of 0.01 mm - 0.05mm.

By mounting the one or more bobbins 401, each bobbing comprising a coil 335, in the at least one cut-out 452 of the probe 300, e.g. by mounting four bobbins 401, each bobbing 401 comprising two protrusions 451, in four times two cut-outs 452, enables the probe 300 to ensure good alignment of the coils 335 with respect to the probe 300.

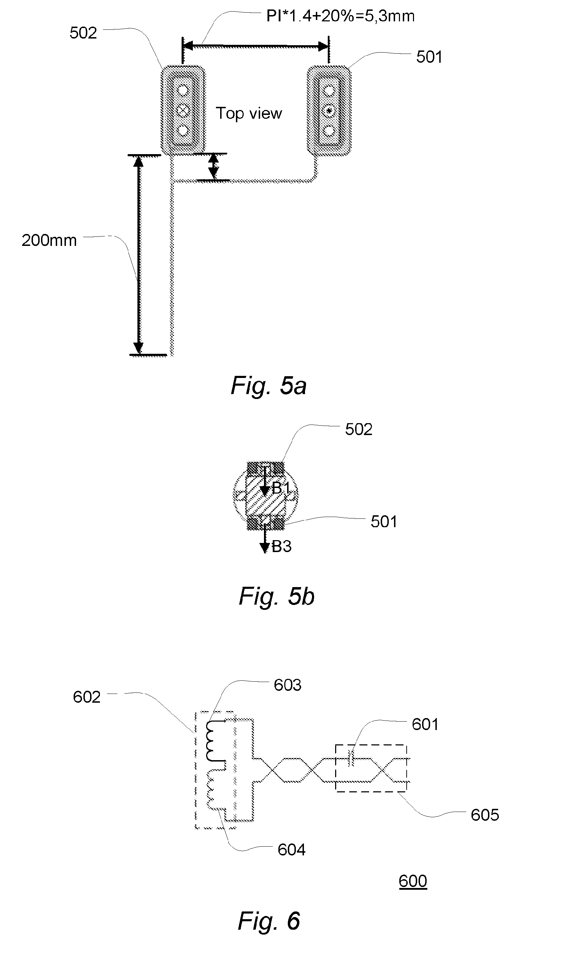

Fig. 5a shows an example of how two coils 335 in two bobbins 401 of a probe 300 may be connected in series e.g. via an electrical connection such as a wire. For example, if the probe of Fig. 4 comprises four coils 335 in respective bobbins 401, then the four coils 335 may be connected two and two in series e.g. via two electrical connections, one electrical connection for each coil pair.

In Fig.5a, a first coil on a first bobbin 501 and a second coil on a second bobbin 502 are shown electrically connected in series. Using Amperes Law (and/or the right hand grip rule), the magnetic field of the first coil may be determined to be directed out of the figure plane and the magnetic field of the second coil may be determined to be directed into the figure plane when a (steady) current is flowing through the respective coils of the two bobbins 501 and 502.

If the second bobbin 502 is placed as coil 335b of figure 3a and the first bobbin 501 is placed as coil 335d of figure 3a, then the first and the second bobbins 501 and 502 may be placed as for example shown in Fig. 5b, and then the magnetic field B3 generated by the first bobbin 501 and the magnetic field B1 generated by the second coil on the second bobbin 502 may add up to Btotal = B1 + B3 thereby increasing the magnetic field generated by the two coils on the bobbins 501 and 502 of probe 300.

Correspondingly, if a second set of two bobbins connected electrically in series are placed with a third bobbin, comprising a third coil generating a third magnetic field B2, as coil 335c of figure 3a and a fourth bobbin, comprising a fourth coil generating a fourth magnetic field B4 as coil 335a, then the magnetic field B2 generated by a third coil on the second bobbin and the magnetic field B4 generated by a fourth coil on the fourth bobbin may add up to B2tal = B2 + B4 thereby increasing the magnetic field generated by the two coils. The described addition of the magnetic vectors an orientation of the magnetic fields as indicated in Fig. 5a. In order to achieve the maximum size of the B2tal magnetic field the B2 and B4 should be aligned to be both parallel and on the same axis. The protrusions and cut-outs in bobbin and the probe ensure this alignment.

Thereby, the probe 300 may provide a first Btotal and a second B2tal magnetic fields, both magnetic fields Btotal and B2tal directed in a direction perpendicular to a longitudinal axis 301 of the probe 300 and Btotal being perpendicular (or substantially perpendicular e.g. 90±0.25 degrees) to B2tal.

The electrical wires connecting two coils in series may be twisted. Additionally, the electrical wires connecting the two coils in series with the probe may be twisted. As an alternative to twisted wires, coaxial cables may be used for one or more of the above named wirings.

In an embodiment, the coils in each of the four coil housings 401 of e.g. figure 4a may be identical. The coils may, for example, have the following values:

| **Parameter** | **Value** | **Unit** |
|---|---|---|
| Turns: | 40 | |
| Wire diameter: | 50 | µm |
| Inductance: | 5.5 | µH |
| Copper resistance: | 3.7 | Ohm |
| Q-factor: | 0.9 | |

In an embodiment, one or more coils 335 (e.g. in respective coil housings 401) may be driven as a series resonance circuit 600 as shown in Fig. 6. For example, if two coils 335 are driven in series, two coils 335 may be included in the resonance circuit 600. The resonance circuit 600 may further comprise a resonance capacitor 601. Further, the resonance circuit may comprise the two coils e.g. 603 and 604.

The capacitor 601 may, for example, be included in a printed circuit board (PCB) 605. The distance between the PCB 605 and the coils 603 and 604 may, for example, be in the order of 120mm thereby providing an effective inductance of 11.6µH and 10.30hm copper resistance of the resonance circuit 600.

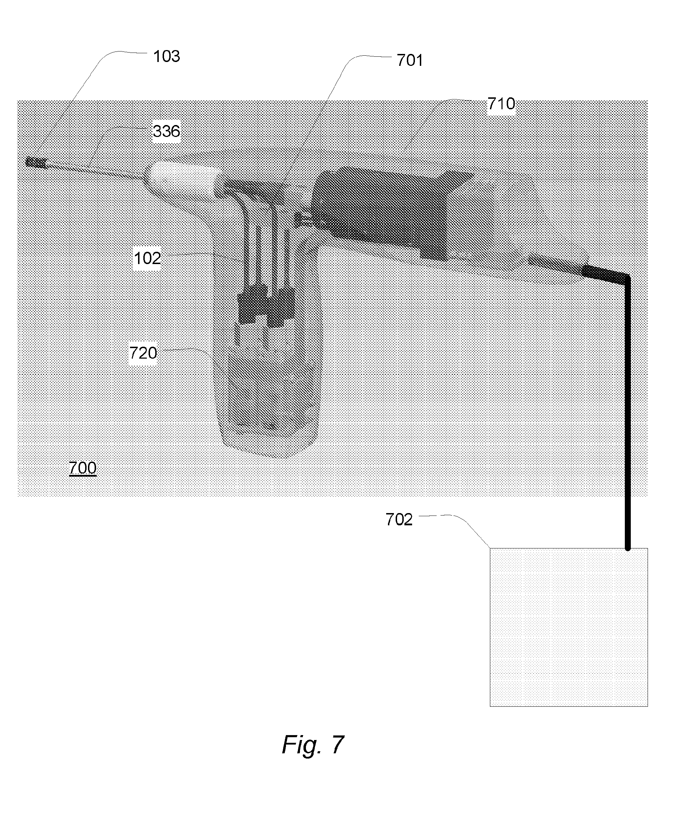

In Fig. 7, the resonance circuit is included in a handheld probe 700. The handheld probe includes a distal light-emitting end portion 103, said distal light-emitting end portion comprising the one or more coils 335 (e.g. four coils) generating one or more magnetic fields (e.g. four magnetic fields B1 - B4). Additionally, the distal light-emitting end portion 103 of the handheld probe 700 may comprise an optical system 116.

Further, the handheld probe 700 may comprise a rod portion 336 which connects the distal light-emitting end portion 103 to a proximal part. The distal light-emitting end portion 103 is rigidly fixed to the rod portion 336 and the rod portion 336 is rigidly fixed to the proximal part 710 e.g. by use of glue or screws or any other means of rigidly fixing.

A handheld probe may, for example, have a volume of 6dm^3. For example, the distal light-emitting end portion 103 and the rod portion 336 may have dimensions of approximately 3mm in diameter and 100mm in length. The proximal part 401 may have dimensions of approximately 100mm in length, 55mm in depth and 100mm in height.

The proximal part 710 may be rigidly fixed in connection with a number of light sources 720, e.g. a number of light sources 720 may be contained in the proximal part 710. For example, if the proximal part 710 contains four laser diodes 720, then the four laser diodes 720 may be contained in the proximal part 710 e.g. by glue to one or more inside walls of the proximal part 710. Alternatively or additionally, the four laser diodes may be fixed to the inside of the proximal part by one or more screws for each of the four laser diodes 710 contained in the proximal part 710.

The handheld probe 700 may further comprise a number of light guides 102 such as one or more optical fibers. In an embodiment, the handheld probing device 700 comprises a light guide 102 for each of the light sources 720 rigidly fixed in connection with the proximal part 710. For example, if the handheld probe 700 comprises four laser diodes 720, then the handheld probe 700 may further comprise four optical fibers 102. One or more of the optical fibers may, for example, be single mode optical fibers. Alternatively or additionally, one or more of the optical fibers may be multimode optical fibers. Alternatively, the proximal part 710 may comprise one or more single mode fibers and one or more multimode fibers.

A first end of each of the light guides 102 may be optically coupled to a rear surface 104 of the optical system 116 in the distal light-emitting end portion 103. For example, the first end of each of the light guides 102 may be glued to the optical system 116 via an optical adhesive. For example, if the handheld probing device comprises four multimode optical fibers, the first end of each of the four multimode optical fibers may be glued to the rear surface 104 of the optical system 116. Alternatively or additionally, the optical system 116 may comprise one or more recesses such that the first end of each light guide 102 may be frictionally coupled to the optical system 116.

A second end of each of the light guides 102 may be optically coupled to a laser diode 720. For example, the second end of each of the light guides 102 may be glued to a respective one of the one or more light sources 720. For example, if the handheld probing device comprises four laser diodes 720 and four multimode optical fibers 102, then the second end of each of the four multimode optical fibers may be glued to a respective one of each of the four laser diodes 720. Alternatively or additionally, the second end of each of the four multimode optical fibers may be frictionally coupled to respective ones of the four laser diodes 720 e.g. via a frictional coupling.

Additionally, the probe 700 may comprise one or more PCBs, each PCB including a resonance capacitor 601. For example, if the probe comprises four coils 335a - 335d, then the probe 700 may comprise two PCBs, each PCB being in connection with two coils. The one or more PCBs may be included in a shielding box 701 e.g. a box made of aluminium. If the probe comprises four coils 335, the shielding box 701 may, for example, comprise the two PCBs. The shielding box 701 may prevent one or more magnetic fields generated by the one or more PCBs in the shielding box 701 from perturbing the magnetic fields generated by the one or more coils 335 and vice versa. A perturbing magnetic field from the electrical circuit in the shielding box 701 could disturb the accuracy of the determination of the position of the probe 700 relative to the external sensors.

If the probe 700 is connected to a signal/data processing unit 702, then the electrical connection between the probe 700 and the signal/data processing unit 702 may be made via a twisted pair cable for each coil pair in the probe 700. Alternatively, the electrical connection between the probe 700 and the signal/data processing unit 702 may be made via a coax cable of a commercially available make, such as the RG-158 coax cable. with one RG-158 coax cable for each coil pair (e.g. sine wave modulating a first coil and a cosine wave modulating a second coil in each of the coil pairs is used for measuring the cross coupling) in the probe 700. Thereby, cross coupling between the two coil pairs in a four coil probe may be minimized which is important to ensure a noise free signal from the probe.

The table below shows an example of the cross coupling levels with either the use of twisted pair cable or RG-158 coax cable for a probe system as shown in 700.

| **Cable type** | **Cross Coupling [dB]** |
|---|---|
| Twisted pair | -53 |
| RG-158 Coax cable | -69 |

A cross coupling level below -60dB may be required in order to calibrate the probe shown in 700. With the above measures and the accurate mounting of the coils in the probe this may be achieved. When calibrating the sine modulated transmitter coil, a small signal may be coupled into the cosine modulated transmitter coil due to cross coupling. The small signal coupled into the cosine modulated transmitter coil may disturb the calibration of the sine modulated transmitter coil. Therefore, it may be required to minimize the cross coupling e.g. by using RG-158 coax cables.

In the above embodiments, the light guides and the image guide have been shown as separate light guides. It will be appreciated that alternatively a single light guide may be used for both directing light to the tip of the probe and for transmitting the reflected light back to the CCD element. Such a combined guide may require an additional beam splitter, and may further have the disadvantage of a reduced signal to noise ratio.

Hence, in the above, a probe of an apparatus has been disclosed which is suitable for obtaining geometrical data of the inner surface of a cavity, such as an ear canal.

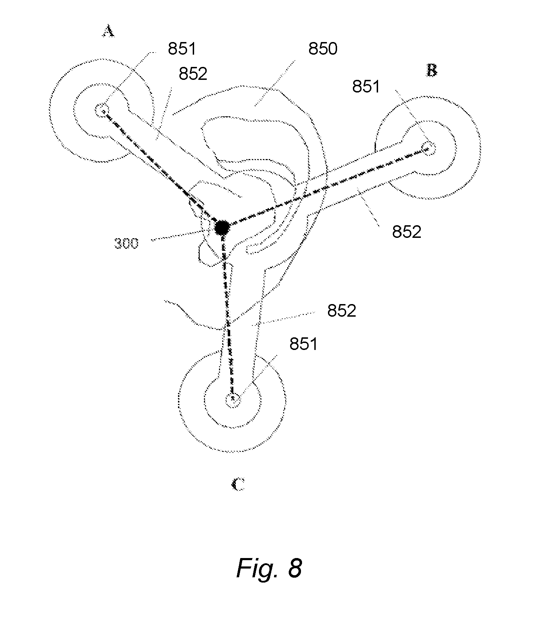

Fig. 8 is a side view showing the human ear and illustrating the use of the apparatus described herein. In use the probe 300 or the distal light-emitting end 103 and part of the rod portion 336 of handheld probe 700 is gently inserted into the ear 850, and magnetic sensors 851 are placed in close relation to the patient's head. Placing the probe in the ear is done while objects in front of the probe are monitored as described above. A real picture may be obtained, and/or the distance to the tympanic membrane is measured as described herein. The picture captured this way is displayed on a monitor, such that the operator may know when the probe is approaching the tympanic membrane. Once the region near the tympanic membrane is reached, the measurements may commence. This may be done while retracting the probe as corresponding values of the distances to the canal wall and the position of the probe are recorded. The recording is continued until the probe reaches the outer regions of the outer ear.

In the example of fig. 8, at each sensor position A, B and C two ore more sensors 851 are located, which are designed to register the magnetic field in each their direction. Through this arrangement the exact location and orientation of the tip of the probe can be determined at any time. In the case shown in fig. 8, the probe 300 or the distal light-emitting end 103 and part of the rod portion 336 of the handheld probe 700 is located inside the canal of a human ear 850, shown schematically in the figure. The three sensor locations are arranged in a fixed construction, which in use is held immobilized relative to the patient's head. In the embodiment shown in fig. 8, the fixed construction comprises a tripod, whereby each of the sensor positions are placed at the outer end of each of the branches 852 of the tripod. The tripod is only schematically indicated in Fig. 8 and will be designed such that it does not cover the ear opening of the user when the sensors A, B and C are mounted to the head. In use, a coil at the tip of the probe is driven at a fixed frequency and by using a lock-in procedure, thereby allowing any noise coming from other magnetic fields in the surroundings to be cancelled out from the sensor signals.

In the embodiments of fig. 3a and fig. 5a, dimensions in the figures are given in millimetres.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A probe and coil fixed thereto for establishing the spatial location of the probe comprising
• a probe body with a first coupling means;
• at least one magnetic field generating device comprising a bobbin and a coil for generating a magnetic field;
• wherein the coil for generating the magnetic field is connected to the bobbin so as to fix a position of the coil for generating a magnetic field relative to the bobbin and wherein the bobbin comprises a second coupling means configured to engage the first coupling means so as to connect the at bobbin and coil to the probe body.

2. A probe and coil according to claim 1, wherein one of the first and second coupling means comprises a protrusion and the other one of the first and second coupling means comprises a hole adapted to receive said protrusion.

3. A probe and coil according to claim 2, wherein the size of the hole adapted to receive the protrusion is identical to or larger than the size of the protrusion.

4. A probe and coil according to claim anyone of claims 1 to 3, wherein the size of a hole in the first coupling means is greater than the size of a corresponding protrusion of the second coupling means and/or the size of a protrusion in the first coupling means are smaller than the size of a corresponding hole in the second coupling means.

5. A probe and coil according to anyone of claims 1 to 4, wherein the first coupling means comprises a first plurality of protrusions and/or a second plurality of holes and wherein the second coupling means comprises the first plurality of corresponding holes and/or the second plurality of corresponding protrusions.

6. A probe and coil according to anyone of claims 1 to 5, wherein the probe comprises at least two bobbins with each their coil adapted to generate respective magnetic fields in substantially the same direction.

7. A method of fixedly position a magnetic generating devices to a probe body wherein the probe comprises a first coupling means and at least one magnetic field generating device wherein the magnetic field generating device comprises a bobbin with a second coupling means and a coil for generating the magnetic field; wherein the method comprises
• connecting the coil for generating a magnetic field to the bobbin so as to fix a position of said coil for generating a magnetic field relative to said bobbin and connecting the at least one magnetic field generating device to the probe body by engaging the second coupling means of the bobbin to the first coupling means of the probe.

8. A system for obtaining geometrical data related to a cavity, the system comprising at least one probe and coil fixedly positioned thereon as claimed in anyone of claims 1 to 7 and the system further comprising at least a plurality of magnetic sensor for detecting the at least one probe.
